(19) **Europäisches Patentamt / European Patent Office / Office européen des brevets**

(11) **EP 1 458 646 B1**

(12) **FASCICULE DE BREVET EUROPEEN**

(45) Date de publication et mention de la délivrance du brevet:
**16.11.2011 Bulletin 2011/46**

(21) Numéro de dépôt: **02799118.1**

(22) Date de dépôt: **24.12.2002**

(51) Int Cl.:
***C01B 33/193*** (2006.01)

(86) Numéro de dépôt international:
**PCT/FR2002/004559**

(87) Numéro de publication internationale:
**WO 2003/055801 (10.07.2003 Gazette 2003/28)**

(54) **Procédé de préparation de silice de précipitation à faible reprise en eau**

Verfahren zur Herstellung einer Fällungskieselsäure mit niedriger Wasseraufnahme

Process for preparation of precipitated silica having a low uptake water

(84) Etats contractants désignés:
**AT BE BG CH CY CZ DE DK EE ES FI FR GB GR IE IT LI LU MC NL PT SE SI SK TR**

(30) Priorité: **26.12.2001 FR 0116881**

(43) Date de publication de la demande:
**22.09.2004 Bulletin 2004/39**

(73) Titulaire: **RHODIA CHIMIE**
**92100 Boulogne Billancourt (FR)**

(72) Inventeurs:
• **VALERO, Rémi**
**F-69005 Lyon (FR)**
• **CHEVALLIER, Yvonick**
**F-69270 Saint-Romain-au-Mont-d'Or (FR)**

(74) Mandataire: **Neyret, Daniel Jean Marie et al**
**Cabinet Lavoix**
**2, place d'Estienne d'Orves**
**75441 Paris Cedex 09 (FR)**

(56) Documents cités:
**EP-A- 0 754 650      EP-A- 0 881 252**
**EP-A1- 0 280 623     WO-A-01/07364**
**WO-A-95/09127        WO-A-95/09128**

**Description**

La présente invention a trait à un procédé de préparation de silice de précipitation, se présentant notamment sous forme de poudres, de granulés ou de billes sensiblement sphériques, et possédant de faibles reprises en eau.

Dans les compositions de type élastomères silicones ou de pâtes de silicones, on utilise depuis de nombreuses années, à titre de charge renforçante, des silices de combustion, c'est à dire des silices obtenues par un procédé consistant à faire réagir à haute température des composés de type tétrachlorosilanes avec de l'hydrogène et de l'oxygène, telles que celles décrites par exemple dans Angewandte Chemie, volume 72, page 744 (1960).

Cependant, de par leur mode d'obtention, les silices de combustion sont généralement onéreuses. De ce fait, dans les applications de renforcement de matrices silicones, on a rapidement cherché à remplacer au moins partiellement ces silices de prix élevé par des silices dites "de précipitation", obtenues par précipitation d'une silice en milieu aqueux à partir d'un précurseur tel qu'un silicate, dans des conditions de pH adéquates. En effet, ces silices sont de moindre coût et elles peuvent présenter les caractéristiques de dispersibilité requises au sein d'une matrice à base de silicone.

Toutefois, il s'avère que, dans le cas le plus général, les silices de précipitation présentent souvent une forte affinité pour l'eau. Cela semble au moins en partie tenir au fait que le mécanisme mis en oeuvre lors de leur précipitation, souvent complexe, met généralement en jeu des phénomènes simultanés, de nucléation, de croissance et de coagulation mal contrôlés, qui conduisent le plus souvent à la formation, à la surface des particules de silice obtenues, de groupements Si-OH avides d'eau.

De façon générale, l'affinité d'une silice vis-à-vis de l'eau se traduit notamment par sa caractéristique dite de "reprise en eau", qui reflète la tendance plus ou moins marquée que présentent les molécules d'eau à s'adsorber sur sa surface. Généralement, la "reprise en eau" d'une silice est mesurée par un test consistant à placer un échantillon de silice préalablement séché, généralement sous un taux d'humidité relative très faible et à une température supérieure à 100°C, dans des conditions d'humidité relatives données et pendant une durée prédéfinie, ce par quoi la silice s'hydrate, ce qui fait passer la masse de l'échantillon d'une valeur initiale m (à l'état séché) jusqu'à une valeur finale (m+dm). La "reprise en eau", qui correspond à la quantité d'eau intégrée à l'échantillon rapportée à la masse de l'échantillon à l'état sec, est égale au rapport dm/m, exprimé en pourcentage.

La "reprise en eau" d'une silice est donc une valeur relative, qui dépend des conditions de séchage et d'hydratation mises en oeuvre dans le test de reprise en eau réalisé. De façon à établir une définition univoque de cette caractéristique au sens de l'invention, dans toute la suite de la description, on ne désignera spécifiquement par "reprise en eau d'une silice" le rapport dm/m exprimé en pourcentage calculé pour un échantillon de la silice soumis aux conditions suivantes lors du test :

- séchage préliminaire : 8 heures, à 105°C;

- hydratation : 24 heures, à 20 °C, et sous une humidité relative de 70%.

Ainsi, pour déterminer, au sens de l'invention, la "reprise en eau" d'une silice, le protocole expérimental mis en oeuvre peut typiquement consister à :

- peser exactement environ 2 g de la silice à tester ;

- sécher pendant 8 heures la silice ainsi pesée dans une étuve réglée à une température de 105°C;

- déterminer la masse m de la silice séchée obtenue à l'issue de ce séchage;

- disposer pendant 24 heures, à 20 °C, la silice séchée obtenue dans un - récipient fermé (par exemple dans un dessicateur) contenant un mélange eau/glycérine avec un ratio massique eau/glycérine de 35/65, de façon à ce que l'humidité relative du milieu fermé soit de 70%;

- déterminer la masse (m+dm) de la silice obtenue suite à ce traitement de 24 heures à 70% d'humidité relative, la mesure de cette masse étant effectuée immédiatement après avoir sorti la silice du dessicateur, de façon à éviter une variation de la masse de la silice sous l'influence du changement d'hygrométrie entre le milieu à 70% d'humidité relative et l'atmosphère du laboratoire.

Au sens de la définition de l'invention, les silices de précipitation les plus usuelles présentent généralement des reprises en eau supérieures à 7 %, et le plus souvent supérieures à 9 %. Ainsi, les reprises en eau des silices de précipitation usuelles sont typiquement de l'ordre de 8 à 10%, et il est rare que des silices de précipitation possèdent des reprises en eau inférieures à 6,5 %.

Une telle affinité pour l'eau est souvent gênante dans le cadre d'une utilisation pour le renforcement de matrices silicones. En effet, il est généralement préférable qu'une silice destinée à renforcer une matrice silicone soit caractérisée par une affinité vis-à-vis de l'eau la plus faible possible. De plus, dans ie cas particulier d'élastomères silicones utilisés à titre d'isolants, dans le câblage électrique par exemple, il est nécessaire que les silices utilisées à titre de charge contiennent le moins d'eau possible, en particulier de façon à ne pas dégrader les propriétés diélectriques du matériau.

En outre, la présence d'eau dans une silice utilisée à titre de charge renforçante dans une matrice silicone, est susceptible de conduire à la formation de bulles dans ladite matrice lorsque celle-ci est mise en forme par extrusion, ce qui constitue un défaut inacceptable dans la pièce extrudée.

Compte tenu de ces éléments, il apparaît donc que les silices de précipitations destinées à être utilisées à titre de charge de renforcement dans des matrices à base de silicone doivent avantageusement posséder non seulement une haute dispersibilité au sein de telles matrices, mais également des valeurs de reprise en eau les plus faibles possible.

Or, les silices de précipitation de l'état de la technique présentent rarement de telles caractéristiques.

De façon à fournir des silices répondant à la double exigence de dispersibilité dans une matrice à base de silicone et de faible affinité vis-à-vis de l'eau, on a donc envisagé de traiter les silices de précipitation connues, à fortes reprises en eau, de façon à diminuer leur affinité pour l'eau.

Dans ce cadre, on a par exemple proposé d'hydrophobiser la surface de silices de précipitation, notamment en mettant en oeuvre des agents de type silanes ou silazanes, comme dans la demande FR 2 356 596. Toutefois, ce type de traitement d'hydrophobisation est généralement relativement coûteux.

Une autre solution dans le cadre du traitement de silices de précipitation à fortes reprises en eau, décrite dans la demande EP 0 280 623, consiste à traiter thermiquement une silice de précipitation à 700-950°C, de préférence en mettant en oeuvre la silice initiale sous la forme de billes dans un four rotatif incliné. Ce procédé est certes moins onéreux que celui de FR 2 356 596, mais il implique néanmoins, là encore, la mise en oeuvre de deux étapes distinctes de préparation et de traitement ultérieur, ce qui se traduit en termes de coûts lors d'une mise en oeuvre industrielle.

Or, les inventeurs ont maintenant découvert qu'une silice de précipitation présentant des caractéristiques de dispersibilité et de reprise en eau particulièrement adaptées à une mise en oeuvre pour le renforcement de matrices à base de silicones peut être réalisée selon un procédé de précipitation de silice, sans qu'il soit nécessaire d'effectuer un traitement ultérieur de la silice de précipitation obtenue.

Plus précisément, les travaux des inventeurs ont permis de mettre en évidence qu'il était possible de réaliser des silices de précipitation présentant une bonne dispersibilité au sein d'une matrice à base de silicone et caractérisées par des reprises en eau inférieure 6%, à condition de réaliser la réaction de précipitation de la silice dans des conditions contrôlées, à la difference de l'enseignement des demandes WO 95/0127 et WO 95/09128 notamment en conduisant la réaction de précipitation en milieu dilué, et en maintenant le pH du milieu réactionnel à une valeur de l'ordre de 7,5 lors de la formation de la silice.

Les résultats obtenus par les inventeurs en ce qui concerne les valeurs de reprise en eau sont particulièrement surprenants. En effet, à l'heure actuelle, les silices les plus intéressantes obtenues à l'issue des procédés de précipitation de l'état de la technique possèdent au minimum des valeurs de reprise en eau comprises entre 6,5 et 7 %.

Sur la base de cette découverte, l'objectif de la présente invention est de fournir un nouveau procédé de préparation de silice de précipitation , simple de mise en oeuvre et économique, et permettant d'obtenir des silices présentant à la fois de bonnes caractéristiques de dispersibilité, en particulier au sein de matrices à base de silicone, et une très faible affinité vis-à-vis de l'eau. Les silices de précipitation otenues par ce procédé sont utilisables à titre d'agents de renforcement dans des matrices polymères à base de silicones, et en particulier dans des matrices élastomères à base de silicones destinées à assurer la fonction d'isolant, par exemple dans l'enrobage de câbles électriques.

Ainsi la présente invention a pour objet un procédé de préparation d'une silice de précipitation à faible reprise en eau, comprenant les étapes successives consistant à :

(a) réaliser un pied de cuve initial comprenant un silicate, la concentration en silicate dans ledit pied de cuve, exprimée en équivalent $SiO_2$, étant inférieure à 15 g/L ;

(b) par addition d'un agent acidifiant, amener le pH du milieu jusqu'à une valeur comprise entre 7 et 8, de préférence jusqu'à une valeur comprise entre 7,2 et 7,8, et avantageusement entre 7,3 et 7,7 (typiquement jusqu'à une valeur sensiblement égale à 7,5) ;

(c) dans le milieu ainsi réalisé, effectuer l'addition simultanée d'un silicate et d'un agent acidifiant, les quantités respectives de silicate et d'agent acidifiant ajoutées au cours du temps étant spécifiquement choisies de façon à ce que, pendant toute la durée de l'addition :

- le pH du milieu réactionnel reste compris entre 7 et 8, et

avantageusement entre 7,2 et 7,8 ; et

- la concentration en silicium dans le milieu, exprimée en équivalent SiO$_2$ reste inférieure ou égale à 35 g/L ;

(d) ajouter un agent acidifiant au milieu obtenu à l'issue de l'étape (c) de façon à porter le milieu à un pH compris entre 3 et 6,5 ; et

(e) filtrer la dispersion aqueuse de silice obtenue, sécher le gâteau de filtration réalisé à l'issu de la filtration, de préférence en le lavant, au préalable,
ce par quoi on obtient ladite silice de précipitation à faible reprise en eau sous forme solide.

Les silicates mis en oeuvre dans les étapes (a) et (c) du procédé de l'invention peuvent être choisis parmi toutes les formes courantes de silicates, et notamment parmi les métasilicates ou les disilicates. Avantageusement, les silicates utilisés selon l'invention sont des silicates alcalins, comme par exemple les silicates de sodium ou de potassium.

De façon particulièrement préférée, le silicate de l'étape (a) est un silicate de sodium, de même que celui ajouté lors de l'étape (c). Le silicate de sodium mis en oeuvre est alors généralement caractérisé par un rapport pondéral SiO$_2$/Na$_2$O compris entre 2 et 4, avantageusement entre 3 et 3,6, ce rapport pondéral SiO$_2$/Na$_2$O étant de préférence compris entre 3,3 et 3,5 (typiquement ce rapport est sensiblement égal à 3,4).

Le pied de cuve de l'étape (a) du procédé de l'invention se présente le plus souvent sous la forme d'une solution aqueuse de silicate dont la concentration est, de façon caractéristique, inférieure ou égale à 15 g/L. Typiquement, la concentration en silicate dans le pied de cuve de l'étape (a), exprimée en équivalent SiO$_2$, est comprise entre 1 g/L et 15 g/L. Cette concentration en silicate dans le pied de cuve de l'étape (a), exprimée en équivalent SiO$_2$ est avantageusement inférieure ou égale à 10 g/L, et de préférence inférieure ou égale à 9 g/L.

Le pied de cuve de l'étape (a) possède généralement un pH de l'ordre de 9 à 13. L'étape (b) du procédé de l'invention consiste spécifiquement à faire diminuer cette valeur du pH, par addition d'un agent acidifiant, de façon à amener le pH du milieu dans la plage de 7 à 8, où les inventeurs ont mis en évidence que la réaction de précipitation de la silice s'effectue de façon optimale. Par "agent acidifiant", on entend, au sens de l'invention, tout composé acide minéral ou organique susceptible de pouvoir mener à une diminution du pH du pied de cuve. Ainsi, à titre d'agent acidifiant, on peut avantageusement mettre en oeuvre un acide minéral tel que l'acide sulfurique, l'acide chlorhydrique ou l'acide nitrique, ou bien encore un acide organique tel que l'acide acétique, l'acide formique ou l'acide carbonique.

De manière avantageuse, aucun électrolyte n'est ajouté au cours du procédé de préparation selon l'invention, en particulier dans l'étape (a). Le terme d'électrolyte s'entend ici dans son acceptation normale, c'est-à-dire qu'il désigne toute substance ionique ou moléculaire qui, lorsqu'elle est en solution, se décompose ou se dissocie pour former des ions ou des particules chargées. On peut citer comme électrolyte un sel du groupe des sels des métaux alcalins et alcalino-terreux, notamment le sel du métal de silicate de départ et de l'agent acidifiant, par exemple ie chiorure de sodium dans le cas de la réaction d'un silicate de sodium avec l'acide chlorhydrique ou, de préférence, le sulfate de sodium dans le cas de la réaction d'un silicate de sodium avec l'acide sulfurique.

L'agent acidifiant mis en oeuvre dans l'étape (b) du procédé de l'invention est de préférence l'acide sulfurique, en particulier lorsque le silicate présent dans le pied de cuve initial est un silicate alcalin. De façon générale, l'agent acidifiant de l'étape (b) est le plus souvent introduit sous la forme d'une solution aqueuse, de préférence diluée, généralement de normalité comprise entre 0,25 N et 8 N. Ainsi, dans l'étape (b), la diminution du pH du milieu peut avantageusement être réalisé par addition d'une solution aqueuse d'acide sulfurique de concentration comprise entre 10 g/L et 350 g/L, et de préférence entre 50 g/L et 250 g/L.

Quelle que soit la nature exacte de l'agent acidifiant de l'étape (b), cet agent acidifiant doit être mis en oeuvre de façon telle que son addition conduise à une diminution du pH du milieu jusqu'à une valeur comprise entre 7 et 8. La quantité d'agent acidifiant à mettre en oeuvre dans ce cadre est généralement déterminée en pratique en mesurant l'évolution du pH au cours de l'addition, l'addition de l'agent acidifiant de l'étape (b) étant poursuivie jusqu'à ce que le pH atteigne la valeur recherchée.

Avantageusement, dans le procédé de l'invention, la diminution de pH de l'étape (b) s'effectue par addition de l'agent acidifiant dans le pied de cuve préalablement porté à une température comprise entre 50 et 100 °C, et de préférence à une température supérieure ou égale à 90°C.

Par ailleurs, l'addition de l'étape (b) s'effectue de préférence de façon progressive, c'est à dire avantageusement, en règle générale, avec une durée d'addition comprise entre 3 et 60 minutes, le plus souvent au moins égale à 5 minutes, et de préférence au moins égale à 10 minutes. Cette durée d'addition est toutefois avantageusement inférieure à 30 minutes.

Selon un mode particulier de réalisation envisageable pour l'étape (b), cette étape peut inclure un processus mûrissement, qui, le cas échéant, est réalisé en laissant le milieu évoluer pendant une durée généralement comprise entre 5 minutes et 30 minutes, de préférence à une température comprise entre 90 et 100°C, étant entendu que, suite à ce mûrissement, le pH milieu réactionnel est adapté si nécessaire, notamment par ajout d'un agent acidifiant, de sorte qu'à l'issue de l'étape (b) le pH du milieu se situe dans la gamme de pH comprise entre 7 et 8, et avantageusement dans les gammes préférentielles précitées.

Suite à l'étape (b), par laquelle le pH du milieu réactionnel est amené dans la zone préférentielle de 7 à 8, et de préférence aux environs de 7,5, l'étape (c) du procédé de l'invention consiste à poursuivre le processus de précipitation de silice, en introduisant du silicate additionnel, et en maintenant spécifiquement le pH du milieu dans la zone comprise entre 7 et 8, de préférence à une valeur sensiblement constante, cette valeur constante étant alors de préférence proche de 7,5, c'est-à-dire généralement comprise entre 7,3 et 7,7.

Pour ce faire, le silicate de l'étape (c) est introduit conjointement à un agent acidifiant, qui s'oppose à l'augmentation de pH qui serait observé en ajoutant le silicate seul. De préférence, l'étape (c) du procédé de l'invention est conduite immédiatement après obtention pour le milieu du pH recherché dans l'étape (b).

L' "addition simultanée" du silicate et de l'agent acidifiant qui est réalisée lors de l'étape (c) consiste avantageusement en une addition continue de silicate dans le milieu au cours de laquelle on mesure le pH du milieu, et pendant laquelle on régule la valeur de ce pH par introduction de l'agent acidifiant, cette introduction de l'agent acidifiant pouvant par exemple être réalisée dès que le pH du milieu devient supérieur à une valeur de contrôle comprise entre 7 et 8, cette valeur de contrôle étant généralement fixée aux alentours de 7,5. Par ce moyen, on arrive à maintenir dans le milieu une valeur sensiblement constante du pH, c'est-à-dire variant avantageusement à +/- 0,2 unité de pH (de préférence à +/- 0,1 unité de pH) autour d'une valeur fixe, généralement comprise entre 7,3 et 7,7.

Alternativement, l'addition simultanée de l'étape (c) peut également consister en une addition continue d'agent acidifiant dans le milieu, le pH étant alors régulé au cours de l'addition par introduction du silicate cette introduction du silicate pouvant là encore être par exemple réalisée dès que le pH du milieu devient inférieur à une valeur de contrôle comprise entre 7 et 8, fixée le plus souvent aux alentours de 7,5. Par ce moyen, on arrive également à maintenir le milieu à un pH sensiblement constant, c'est-à-dire variant avantageusement à +/- 0,2 unité de pH (de préférence à +/- 0,1 unité de pH) autour d'une valeur fixe, généralement comprise entre 7,3 et 7,7.

Selon encore un autre mode de mise en oeuvre envisageable, l'addition simultanée de l'étape (c) peut également consister en une addition continue à la fois d'agent acidifiant et de silicate, avec des concentrations et des débits calculés de façon à ce que, durant toute la durée de l'addition, le pH du milieu reste compris entre 7 et 8, et de préférence entre 7,2 et 7,8. Dans ce cas, le pH du milieu a généralement tendance à évoluer au cours de l'étape (c), tout en restant dans la gamme précitée, mais il peut, dans certains cas, demeurer sensiblement égal à une valeur constante, avantageusement de l'ordre de 7,5. Dans ce cadre, on préfère généralement que, tout au long de l'étape (c), les débits instantanés correspondant à la quantité de fonctions silicates (exprimées en équivalent molaire de NaOH) introduites par seconde (notée $d_S$), et la quantité de fonctions acides (en moles) introduites par seconde (notée $d_A$), soient tels que le rapport $d_S/d_A$ reste en permanence compris entre 1,01 et 1,09, et de préférence entre 1,02 et 1,07.

Quel que soit le mode exact de mise en oeuvre de l'étape (c), le silicate et l'agent acidifiant utilisés sont le plus souvent identiques à ceux mis en oeuvre dans les étapes (a) et (b). Ainsi, le silicate de l'étape (c) est de préférence un silicate alcalin, avantageusement un silicate de sodium, et l'agent acidifiant est préférentiellement un acide minéral fort, le plus souvent l'acide sulfurique.

Dans la mesure où, lors de l'addition simultanée de l'étape (c), la concentration en silicium dans le milieu (exprimée en équivalent $SiO_2$ doit, de façon caractéristique, être maintenue inférieure ou égale à 35 g/L, le silicate introduit dans le milieu réactionnel lors de l'étape (c) est généralement sous la forme d'une solution aqueuse diluée, c'est à dire de concentration, exprimée en équivalent $SiO_2$, avantageusement comprise entre 10 g/L et 360 g/L, de préférence inférieure à 300 g/L et avantageusement inférieure à 270 g/L, et ce tout particulièrement lorsqu'on utilise des silicates alcalins tels que les silicates de sodium. De la même façon, l'agent acidifiant est le plus souvent sous la forme d'une solution aqueuse diluée, qui possède généralement une normalité comprise entre 0,25 N et 8 N, et de préférence entre 0,5 N et 4 N. Ainsi, dans le cas spécifique de l'utilisation d'une solution aqueuse d'acide sulfurique à titre d'agent acidifiant de l'étape (c), par exemple, la concentration de la solution est avantageusement comprise entre 25 g/L et 380 g/L, et de préférence entre 50 g/L et 300 g/L.

Il est à souligner que, compte tenu de la mise en oeuvre de concentrations diluées dans le milieu de précipitation des silices du procédé selon l'invention, les concentrations en sels dans ce milieu, notamment liées à la réaction du silicate et de l'agent acidifiant , sont façon caractéristique, extrêmement faible, ce qui se traduit par une faible force ionique au sein du milieu de précipitation mis en oeuvre.

Sans vouloir être lié en aucune façon à une théorie particulière, il semble pouvoir être avancé que le contrôle du pH et des concentrations mis en oeuvre selon l'invention permet de minimiser la formation de groupements de surface SiOH qui sont généralement formés en nombre important dans les procédés de l'état de la technique qui ne mettent pas en oeuvre un tel contrôle. L'invention permet ainsi d'obtenir des silices de précipitation présentant des reprises en eau extrêmement réduites.

De façon à améliorer encore le contrôle de la formation de la silice, les inventeurs ont mis en évidence qu'il était particulièrement avantageux de réaliser l'addition simultanée de l'étape (c), avec des débits de silicate et d'agent acidifiant relativement faibles, c'est à dire, le plus souvent, avec une durée d'addition de l'étape (c) de préférence comprise entre 15 et 300 minutes, et de préférence entre 30 et 100 minutes. De telles durées d'addition mènent en effet généralement à l'obtention de particules de silices présentant des taux de groupements Si-OH en surface extrêmement réduits.

De façon générale, l'étape (c) du procédé de l'invention est avantageusement conduite sous agitation et de préférence à une température comprise entre 50 et 100°C, et généralement à la même température que l'addition de l'étape (b). Ainsi, la température de mise en oeuvre de l'étape (c) peut avantageusement être comprise entre 90 et 100°C, et elle est de préférence de l'ordre de 95°C.

Selon une variante particulière du procédé de l'invention, on peut introduire dans le milieu réactionnel, au cours de l'étape (c), de préférence à la fin de cette étape (c'est à dire typiquement durant la période correspondant au dernier quart de cette étape, généralement au cours des 5 à 15 dernières minutes de cette étape) un composé à base d'aluminium, de préférence un sel de nature acide comme un sulfate d'aluminium, ou, alternativement, un composé de nature basique tel qu'un aluminate de sodium. L'addition de ce composé à base d'aluminium peut, entre autres, mener à une amélioration des caractéristiques de reprise en eau de la silice obtenue, et, dans le cadre spécifique de la mise en oeuvre d'acide sulfurique à titre d'agent acidifiant, elle permet de diminuer la teneur en sulfate dans la silice obtenue à l'issue du procédé de l'invention. La quantité de composé d'aluminium introduite dans ce cadre est généralement telle qu'au sein du milieu réactionnel, le ratio $Al/SiO_2$ est compris entre 0,1 et 1 % en masse, ce ratio étant de préférence au plus égal à 0,6%, et de préférence inférieur ou égal à 0,5%.

Quel que soit le mode exact de réalisation de l'étape (c), à l'issue de cette étape, le milieu réactionnel est spécifiquement à un pH compris entre 7 et 8, et de préférence de l'ordre de 7,5.

En fonction des applications envisagée pour la silice préparée selon le procédé de l'invention, l'étape (d) d'acidification du milieu dans la zone de pH de 3 à 6,5 peut être modulée par la quantité d'agent acidifiant additionné. De préférence, le pH du milieu atteint à l'issue de l'étape (d) est compris entre 3,5 et 5,5.

L'agent acidifiant de l'étape (d) peut indifféremment être identique ou différent de celui ou ceux mis en oeuvre dans les étapes (b) et (c). De préférence, cet agent acidifiant de l'étape (d) est introduit dans le milieu sous la forme d'une solution aqueuse de normalité comprise entre 0,25 et 8 N. Avantageusement, il s'agit d'une solution aqueuse d'acide sulfurique, généralement à une concentration comprise entre 25 et 380 g/L le cas échéant.

Le plus souvent, la température à laquelle est conduite l'éventuelle étape (d) est comprise entre 50 et 100°C, et elle peut ainsi être identique à celle mise en oeuvre lors de l'étape (c) précédente.

Généralement, on préfère d'ailleurs que l'ensemble des étapes (a), (b), (c) et (d) du procédé soient conduites à une température de 90 et 100°C, et avantageusement à une température comprise entre 93 et 97°C, et encore plus avantageusement à une température sensiblement égale à 95°C tout au long du procédé.

Selon une variante avantageuse du procédé de l'invention, les dispersions aqueuses de silice obtenues à l'issue des étapes (c) et (d) peuvent être soumises à une étape de mûrissement, généralement conduite, ie cas échéant, en laissant le milieu, de préférence sous agitation, à une température comprise entre 90 et 100 °C, pendant une durée qui peut être avantageusement comprise entre 15 minutes et 240 minutes, et de préférence pendant une durée supérieure à 30 minutes, la température lors du mûrissement étant préférentiellement sensiblement constante (le cas échéant, avantageusement sensiblement égale à 95°C), ou bien croissante (généralement par pallier(s) le cas échéant) dans la gamme de température allant de 90 à 100°C.

Il est à souligner que l'addition d'un composé d'aluminium, notamment de type sulfate d'aluminium, qui est envisageable à la fin de l'étape (c) peut également être conduite au cours de l'étape (d), ou bien encore au cours de l'étape de mûrissement ultérieure, lorsque cette étape est mise en oeuvre. Ainsi, de façon générale, cette addition d'un composé à base d'aluminium dans le milieu peut avoir lieu entre l'étape (c) et l'étape (e).

L'étape (e) du procédé de l'invention consiste, de façon globale, à récupérer une silice sous forme solide à partir de la dispersion obtenue à l'issue des étapes précédentes.

Le plus souvent, lors de cette étape (e), la dispersion obtenue à l'issue de l'étape (d) et de l'éventuelle étape de mûrissement ultérieure, est soumise à une filtration sur filtre presse ou à une filtration sous vide en utilisant un filtre rotatif, un filtre à bande, ou encore un filtre plan, cette filtration conduisant à l'obtention d'un "gâteau de silice", c'est à dire une bouillie de silice à teneur en eau relativement importante. Le gâteau de silice obtenu est alors le plus souvent soumis à une étape de lavage, généralement par de l'eau, de façon à réduire sa teneur en sels, et il est ensuite soumis à un séchage, notamment par une atomisation adaptée, et par exemple à l'aide d'un atomiseur à turbines, à buse, à pression liquide ou à deux fluides.

Dans ce cadre, on délite généralement le gâteau de silice au préalable, de façon à former une bouillie de silice de viscosité suffisamment faible, pour assurer son pompage jusqu'à l'atomiseur. Cette bouillie présente de préférence une perte au feu à 1000°C inférieure ou égale à 82% en masse.

Le cas échéant, l'opération de délitage peut par exemple être réalisée en faisant passer le gâteau de filtration dans un broyeur de type moulin colloïdal ou à billes, ou bien encore en le soumettant à une désagrégation sous ultrasons. En particulier dans le cadre d'un gâteau de silice obtenu par filtration sur filtre presse, il est avantageux de mettre en oeuvre lors de l'étape de délitage un processus d'agitation sous cisaillement élevé, par exemple de type contrarotatif. On peut envisager l'addition d'un composé à base d'aluminium pour faciliter l'opération de délitage.

Le plus souvent, la bouillie de faible viscosité issue d'un tel délitage se présente sous la forme d'une dispersion aqueuse de silice qui est directement pompé vers un atomiseur pour l'étape (e). Toutefois, on peut également envisager de conduire l'étape (e) ultérieurement, notamment sur un autre site d'exploitation. En effet, la bouillie de faible viscosité obtenue suite au délitage constitue dans le cas général une composition aqueuse de silice facilement transportable et stockable, et qui peut à tout moment, sous l'effet d'une contrainte mécanique suffisante, être mise sous la forme d'une dispersion de silice de viscosité suffisamment fluide pour être pompée, puis séchée par atomisation. Cette composition aqueuse de silice spécifique constitue un objet particulier de la présente invention.

En particulier lorsqu'elle est obtenue par séchage d'un gâteau de filtration délité de perte au feu inférieure à 82%, la silice précipitée obtenue selon le procédé de l'invention peut se présenter sous la forme de billes sensiblement sphériques, possédant un diamètre moyen se situant de préférence entre 80 et 350 $\mu$m, avantageusement entre 150 et 280 $\mu$m. Cette taille moyenne des billes est déterminée selon la norme NF X 11507 (décembre 1970) par tamisage à sec et détermination du diamètre correspondant à un refus cumulé à 50 %. La densité de remplissage à l'état tassé (DRT) de ce type de billes, mesurée selon la norme NF T 30 042, est, en général, d'au moins 0,2 et elle est typiquement comprise entre 0,25 et 0,3. Par ailleurs, ces billes présentent habituellement un volume poreux total d'au moins 3 cm$^3$/g, avantageusement compris entre 3,5 et 4,5 cm$^3$/g, ce volume poreux étant mesuré par porosimétrie au mercure (les échantillons testés étant séchés pendant 2 heures à 200°C sous étuve, puis dégazés immédiatement sous vide, dans les 5 minutes suivant la sortie de l'étuve), et les diamètres de pores étant calculés par la relation de WASHBURN avec un angle de contact théta égal à 140°C et une tension superficielle gamma égale à 484 Dynes/cm (porosimètre MICROMERITICS 9300).

Ce type de bille peut être soumis à un broyage ultérieur, de façon à obtenir la silice de précipitation sous la forme d'une poudre au sein de laquelle les particules peuvent présenter, en fonction de l'intensité du broyage, une taille moyenne comprise entre 3 et 15 microns.

De façon plus générale, en fonction du type de séchage réalisé, les silices de précipitation obtenues à l'issue de l'étape (e) peuvent se présenter sous la forme de poudres de particules isotropes ou anisotropes, présentant des dimensions moyennes qui peuvent en règle générale être comprises entre 3 et 350 microns. La densité de remplissage à l'état tassé (DRT) de ces poudres, mesurée selon la norme NF T 30 042, est généralement comprise entre 0,1 et 0,3. Par ailleurs, ces poudres présentent habituellement un volume poreux total, tel que défini ci-avant pour les billes avantageusement compris entre 3 et 6 cm$^3$/g.

Dans le cadre de la préparation de silice spécifiquement destinée à une utilisation dans le renfort de matrices à base de silicone, on préfère que la silice réalisée se présente sous la forme de particules de taille moyenne comprise entre 3 et 30 microns, et de préférence entre 5 et 15 microns. De façon à atteindre de telles granulométries, on peut utiliser tout type de silice obtenue selon le procédé de l'invention, le soumettre à une éventuelle étape de broyage, puis, si nécessaire, séparer les différentes granulométries présentes au sein de la silice obtenue, par exemple par mise en oeuvre de tamis vibreurs possédant des tailles de mailles adaptées, les particules de taille trop importante pouvant être récupérées et renvoyées au broyage.

Les silices séchées obtenues à l'issue de l'étape (e) peuvent également être soumises à une étape d'agglomération, notamment par compression directe, par une granulation en voie humide (c'est-à-dire avec utilisation d'un liant tel que l'eau), par extrusion et, de préférence, par compactage à sec. Lorsque l'on met en oeuvre cette dernière technique, il peut s'avérer avantageux, avant de procéder au compactage, de désaérer (opération aussi appelée pré-densification ou dégazage) les produits pulvérulents de manière à éliminer l'air inclus dans ceux-ci et assurer un compactage plus régulier. A l'issue de l'étape d'agglomération, les produits peuvent être calibrés à une taille désirée, par exemple par tamisage, puis conditionnés pour une utilisation ultérieure. La silice précipitée compactée susceptible d'être obtenue selon ce mode de réalisation particulier de l'invention se présente avantageusement sous la forme de granulés. Le cas échéant, ces granulés peuvent se présenter sous des formes les plus diverses. A titre d'exemple, on peut notamment citer les formes sphérique, cylindrique, parallélépipédique, de pastille, de plaquette, de boulette, d'extrudé à section circulaire ou polylobée. Les dimensions moyennes de ces granulés sont préférentiellement comprises entre 2 mm et 20 mm. Par ailleurs, la densité de remplissage à l'état tassé desdits granulés, mesurée selon la norme NF T 30 042, est en général d'au moins 0,15 et peut aller jusqu'à 0,35, et ces granulés présentent généralement un volume poreux total compris entre 2,5 et 4,5 cm$^3$/g

Quelle que soit la forme sous laquelle elles se présentent, les silices de précipitation obtenues à l'issu du procédé de l'invention possèdent généralement une très faible valeur de reprise en eau au sens de l'invention, associée notamment à une surface spécifique importante et une faible densité, qui leur confère une haute dispersibilité notamment au sein de matrices élastomères à base de silicone, ce qui les rend particulièrement adaptées à une utilisation à titre de charge de renforcement dans une matrice à base de silicone.

Ainsi, le plus souvent, le procédé de l'invention conduit à l'obtention de silices de précipitation possédant une reprise en eau inférieure à 6%. Rappelons que la reprise en eau à laquelle il est fait référence ici est la reprise en eau au sens de l'invention, mesuré selon le test de séchage/hydration défini précédemment. Dans la plupart des cas, la reprise en eau des silices obtenues selon le procédé de l'invention est inférieure ou égale à 5,9%, et généralement inférieure à 5,8%. Ainsi, de façon particulièrement avantageuse, la reprise en eau des silices obtenues selon le procédé de l'invention peut être inférieure ou égaie à 5,7%, et même inférieure ou égale à 5,5%. Avantageusement, elle est inférieure ou égale à 5,4%, voire inférieure ou égale à 5,3%. Dans le cas le plus général, elle reste néanmoins le plus souvent supérieure à 4%, et généralement supérieure à 4,5%.

En plus de cette faible reprise en eau, les silices obtenues selon le procédé de l'invention sont généralement telles qu'après un traitement thermique à 105°C pendant 8 heures, ils présentent une teneur en eau résiduelle généralement comprise entre 2 et 4 % en masse par rapport à la masse totale de l'échantillon, de préférence inférieure à 3% en masse par rapport à la masse totale de l'échantillon, et avantageusement inférieure à 2,7 % en masse par rapport à la masse totale de l'échantillon.

Par ailleurs, les silices de précipitations obtenues selon le procédé de l'invention possèdent généralement une surface spécifique BET, telle que mesurée selon la méthode BRUNAUER-EMMET-TELLER décrite dans *the* Journal of the American Chemical Society, vol. 60, page 309 (février 1938), supérieure à 100 m$^2$/g, et de préférence au moins égale à 120 m$^2$/g. Cette surface spécifique BET est avantageusement au moins égale à 150 m$^2$/g, mais elle reste généralement inférieure ou égale à 200 m$^2$/g. Ainsi, elle est typiquement comprise entre 120 et 185 m$^2$/g.

Les silices obtenues selon le procédé de l'invention peuvent également être caractérisées par une surface spécifique dite CTAB, determinée par adsorption de bromure de cétyl-triméthylammonium à pH=9, selon la norme NFT 45007 (novembre 1987). Généralement, la surface spécifique CTAB des silices obtenues selon le procédé de l'invention est comprise entre 100 et 200 m$^2$/g, et elle est avantageusement au moins égale à 110 m$^2$/g , et de préférence au moins égale à 130 m$^2$/g. Elle peut ainsi être typiquement comprise entre 120 et 185 m$^2$/g.

Par ailleurs, les silices obtenues selon le procédé de l'invention sont généralement caractérisées par une prise d'huile DOP, déterminée selon la norme NFT 30-022 (mars 1953), en mettant en oeuvre le dioctylphtalate, comprise entre 150 ml/100g et 300 m1/100g, et de préférence supérieure à 200 ml/100g.

Les silices de précipitation issues du procédé de l'invention contiennent généralement, au moins à l'état de traces, un sel résultant de l'action des agents acidifiant mis en oeuvre sur les silicates utilisés. Ainsi, lorsque le procédé de l'invention met spécifiquement en oeuvre un silicate alcalin en tant que précurseur de la silice et l'acide sulfurique à titre d'agent acidifiant, les silices de précipitation contiennent un sulfate alcalin. Généralement, la teneur en sulfate alcalin dans les silices ainsi obtenues est relativement faible, le plus souvent tel que la masse des ions sulfates présents représente généralement au plus 0,5 % en masse par rapport à la masse totale du matériau sec (typiquement entre 0,1 et 0,5 % en masse par rapport à la masse totale du matériau sec). Cette teneur en ions sulfate peut encore être abaissée en effectuant une filtration suivie d'un lavage poussé du gâteau de silice lors de l'étape (e) du procédé. Dans ce cas, la masse des ions sulfates présents représente généralement entre 0,05 et 0,4 % en masse par rapport à la masse totale du matériau sec, et elle est avantageusement inférieure à 0,3 %, de préférence inférieure à 0,2%, et encore plus avantageusement inférieure à 0,1% en masse par rapport à la masse totale du matériau sec.

Dans le cadre de la mise en oeuvre d'un silicate de sodium à titre de précurseur de la silice dans le procédé de l'invention, la silice obtenue à l'issu du procédé contient spécifiquement des ions sodium, quel que soit la nature des agents acidifiants mis en oeüvre. Généralement, la teneur en sodium résiduel au sein des silices ainsi obtenues est comprise entre 300 et 1500 ppm, cette teneur pouvant être inférieure ou égale à 1000 ppm, et avantageusement inférieure ou égale à 800 ppm. Cette teneur en sodium est mesurée par spectroémission de flamme d'un échantillon de la silice de masse déterminée, dissous dans de l'acide fluorhydrique.

Par ailleurs, les silices issues du procédé de l'invention sont caractérisées par un pH, mesuré selon la norme ISO 787/9, (pH d'une suspension à 5 % en masse de la silice dans l'eau), généralement compris entre 4,5 et 7, et de préférence entre 4,8 et 6.

Les silices de précipitation obtenues selon le procédé de la présente invention présentent une très bonne aptitude à la dispersion et elles sont particulièrement adaptées pour une utilisation à titre de charge renforçante dans des matrices à base de silicones, auxquelles elles confèrent de bonnes propriétés rhéologiques, tout en leur procurant des propriétés mécaniques très satisfaisantes.

Les silices préparées par le procédé selon l'invention trouvent ainsi une application particulièrement intéressante dans le renforcement de matrices élastomères à base de silicones, telles que, par exemple les matrices élastomères vulcanisables à froid ou à chaud.

Les silices obtenues selon le procédé de l'invention sont particulièrement adaptées à l'utilisation comme charge de compositions organosiliciques.

La nature des compositions organosiliciques pouvant être renforcée par les silices obtenues selon le procédé de l'invention n'est pas critique. De manière générale, Ces compositions organosiliciques peuvent être de nature élastomères ou pâteuses.

Dans le cas des compositions élastomères, on met généralement en oeuvre un polymère organosilicique vulcanisable tel que, en désignant par R les radicaux de nature hydrocarbonée liés aux atomes de silicium, le rapport entre le nombre total de radicaux R et le nombre total d'atomes de silicium, est compris entre 0,5 et 3. Dans la constitution du polymère organosilicique, les autres valences disponibles du silicium sont reliées à des hétéro-atomes tels que l'oxygène ou l'azote, ou bien à des radicaux hydrocarbonés multivalents.

Préférentiellement les compositions organosiliciques chargées par les silices obtenues selon le procédé de l'invention sont des compositions organopolysiloxaniques dans lesquelles l'organopolysiloxane peut être linéaire ou ramifié, et éventuellement comporter en plus des radicaux hydrocarbonés des groupements réactifs comme par exemple des groupements hydroxyles, des groupements hydrolysables, des groupements alkényle, ou des atomes d'hydrogène.

Plus précisément les organopolysiloxanes constituants principaux de ces compositions sont constitués de motifs siloxaniques de formule générale.

$$R_n SiO \frac{4-n}{2} \qquad (I)$$

éventuellement associés à des motifs siloxaniques de formule :

$$Z_x R_y SiO \frac{4-x-y}{2} \qquad (II)$$

Dans ces formules les divers symboles ont la signification suivante :

- R représente un groupement de nature hydrocarbonée non hydrolysable, ce radical pouvant être :

- un radical alcoyle ou halogènoalkyle ayant de 1 à 5 atomes de carbone et comportant de 1 à 6 atomes de chlore et/ou de fluor ;

- des radicaux cycloalcoyles et halogènocycloalcoyles possédant de 3 à 8 atomes de carbone et contenant de 1 à 4 atomes de chlore et/ou de fluor ;

- des radicaux aryles et halogénoaryles ayant de 6 à 8 atomes de carbone et contenant de 1 à 4 atomes de chlore et/ou de fluor ;

- des radicaux cyanolcoyles ayant de 3 à 4 atomes de carbonate.

- Z représente un atome d'hydrogène, un groupement alcényle, un groupement hydroxyle, un atome hydrolysable, un groupement hydrolysable.

- n représente un nombre entier égal à 0, 1, 2 ou 3

- x représente un nombre entier égal à 0, 1, 2 ou 3

- y représente un nombre entier inférieur ou égal à 2.

A titre illustratif, on peut citer parmi les radicaux organiques R, directement liés aux atomes de silicium les groupes méthyle ; éthyle ; propyle ; isopropyle ; butyle ; isobutyl ; α-pentyle ; t-butyle ; chlorométhyle ; dichlorométhyle ; α-chloroéthyle ; α,β-dichloroéthyle ; fluorométhyle ; difluorométhyle ; α-β-difluoroéthyle ; tri-fluoro-3,3,3 propyle ; tri-fluoro cyclopropyle ; cyclopropyle ; trifluoro-4,4,4, butyle ; heptafluoro-3,3,4,4,5,5 pentyle ; β-cyanoéthyle ; γ-cyanopropyle ; phenyle ;p-chlorophényle ; m-chlorophényle ; dichloro-3,5 phényle ; trichlorophényle ; tétrachlorophényle ; o-, p-, ou m-totyle ; α,α,α-trifluorotolyle ; xylyles comme diméthyl-2,3 phényle ; diméthyl-3,4 phényle.

Préférentiellement les radicaux organiques liés aux atomes de silicium sont des radicaux méthyle, phényle, ou vinyle, ces radicaux pouvant être éventuellement halogénés ou bien encore des radicaux cyanoalkyle.

Les symboles Z représente avantageusement des atomes d'hydrogène ou de chlore, des groupements vinyles, des groupements hydroxyles ou des groupements hydrolysables tels que des radicaux amino, amido, aminoxy, oxime, alcoxy, alcoxyalcoxy, alkenyloxy, acyloxy.

La nature de l'organopolysiloxane, et donc les rapports entre les motifs siloxaniques (1) et (11), et la répartition de ceux-ci, est généralement choisie en fonction de l'application envisagée et en fonction du traitement de vulcanisation qui sera effectuée sur la composition.

Il peut ainsi s'agir de compositions vulcanisables à température élevée sous l'action de peroxydes organiques tels que le peroxyde de cichloro-2,4 benzoyle, le peroxyde de benzoyle, le perbenzoate de t-butyle, le peroxyde de cumyle, le peroxyde de di-t-butyle.

L'organopolysiloxane entrant dans de telles compositions est alors constitué essentiellement de motifs siloxaniques (I) et ne contient pas de groupes ou d'atomes hydrolysables.

Les polyméthylpolysiloxanes terminés par des groupements triméthylsilyles représentent un exemple particulièrement important de cette catégorie sur le plan industriel.

La vulcanisation peut être également effectuée à température ambiante ou à température modérée par création de réticulations entre des groupements vinylsilylés et des groupements hydrogénosilylés, la réaction d'hydrosilylation étant effectuée en présence de catalyseurs tels que les dérivés du platine. Les organopotysiloxanes mis en oeuvre ne contiennent pas alors d'atomes ou de groupements hydrolysables.

La vulcanisation peut être effectuée sous l'action de l'humidité. Les organopolysiloxanes contenus dans les compositions de ce type contiennent des atomes ou des groupements hydrolysables tels que précédemment définis. Les motifs siloxaniques (11) contenant de tels groupements représentent au plus 15 % en poids de la masse totale de l'organopolysiloxane mis en oeuvre. Les compositions organopolysiloxaniques de ce type contiennent généralement des catalyseurs tels que le sel d'étain.

La vulcanisation peut enfin être effectuée en présence d'agents de réticulation. Les organopolysiloxanes entrant dans ces compositions sont en général des polysiloxanes linéaires ramifiées ou réticulées constituées de motifs (1) et (11) dans lesquels Z est un groupement hydroxyle et où x est au moins égal à 1. L'agent de réticulation peut être un silane polyfonctionnel tels que le méthyltriacétoxysilane, l'isopropyltriacetoxysilane, le vinyltriacétoxysilane, le méthyltris (diéthylaminoxy)silane. Divers autres composés tels que les silicates peuvent être utilisés comme agents de réticulation.

Les compositions organosiliciques contiennent de 5 à 50 % et de préférence de 10 à 40 % de silices de précipitation éventuellement traitées telles que précédemment définies. Dans le cas des pâtes silicones, la proportion en silice obtenue par le procédé de l'invention sera généralement comprise entre 3 et 20 %.

En outre, en plus des polysiloxanes, de la silice précipitée éventuellement traitée, des agents de réticulation et des catalyseurs de réticulation, les compositions peuvent contenir des charges usuelles telles que du quartz pulvérisé, de la terre de diatomées, du talc, du noir du carbone, du carbonate. Les compositions peuvent en outre contenir des adjuvants divers usuels comme des agents antistructure, des stabilisants thermiques, des agents thixotropiques, des pigment, des inhibiteurs de corrosion.

Les agents antistructure, connus également sous la dénomination de plastifiants, sont en général de nature organosilicique et sont introduits à raison de 0 à 20 parties pour 100 parties de gomme organosilicique. Ils permettent d'éviter le durcissement des compositions lors du stockage. Parmi les agents antistructure on peut citer les silanes à groupements hydrolysables, ou des huiles diorganopolysiloxaniques hydroxylées ou alcoxylées de faible poids moléculaire. De telles compositions sont par exemple décrites dans le brevet français FR-A-1.111.969.

Parmi les stabilisants thermiques qui sont bien connus de l'homme de l'art on peut citer les sels, les oxydes et les hydroxydes de fer, de cérium ou de manganèse. Ces additifs qui peuvent être utilisés seuls ou en mélange sont en général introduits à raison de 0,01 à 5 % par rapport au poids de la gomme organopolysiloxanique mise en oeuvre.

Les compositions organopolysiloxaniques sont préparées en mélangeant les divers ingrédients de la composition tels que précédemment décrits. Le mélange peut être effectué à température ambiante ou à chaud.

Les matrices à base de silicone peuvent être mises en forme par extrusion avant d'être réticulées. Les silices obtenues selon le procédé de l'invention se révèlent particulièrement utiles à titre de charge de renforcement dans une telle matrice à base de silicone mise en forme par extrusion, où leur faible teneur en eau limite la formation de bulles.

En plus de leurs applications à titre de charges dans des matrices à base de silicones, les silices de faible reprise en eau issues du procédé de la présente invention peuvent aussi être avantageusement utilisées à titre de charge de renforcement dans des matrices à base de polymères organiques, et en particulier dans des matrices à base d'un ou plusieurs élastomères, naturels ou synthétiques, et notamment dans des matrices à base de caoutchouc, et plus particulièrement à base de caoutchoucs naturels ou synthétiques, de type SBR ou caoutchouc butyle en particulier. En effet, les silices obtenues selon le procédé de l'invention de bonnes qualité de dispersibilité et de renfort au sein de matrices polymères et élastomères, où elles permettent notamment d'augmenter la résistance à l'abrasion, ce qui peut s'avérer intéressant dans le cadre de la constitution de pneumatiques.

Dans le cadre du renfort des matrices élastomères, les inventeurs ont par ailleurs mis en évidence que les silices obtenues selon le procédé de la présente invention sont particulièrement adaptées pour réaliser le renfort de matrices transparentes ou translucides, telles que celles utilisées en particulier pour la confection de pièces en caoutchouc transparentes ou translucides constitutives de chaussures (semelles par exemple), où elles affectent beaucoup moins les caractéristiques de transparence ou de translucidité que la plupart des autres silices actuellement connues mises en oeuvre dans ce cadre.

Les silices de faible reprise en eau obtenues par le procédé de la présente invention peuvent également être avantageusement utilisées à titre d'agents épaississant au sein de milieux organiques ou aqueux, de préférence au sein de milieux aqueux, et notamment au sein de pâtes dentifrices.

Par ailleurs, les silices obtenues selon le procédé de l'invention peuvent s'avérer utiles dans de nombreux autres domaines usuels d'utilisation des silices de précipitation, par exemple pour la fabrication de peintures ou de papiers. Elles se révèlent particulièrement intéressantes à titre de support dans des compositions alimentaires ou cosmétiques.

De par leur faible teneur en sels, les silices obtenues selon le procédé de la présente invention sont par ailleurs des silices particulièrement bien adaptées dans le domaine de la galénique. Ainsi, les silices de la présente invention sont particulièrement adaptées à titre de charges, de supports et/ou d'excipients au sein de compositions pharmaceutiques.

L'objet et les avantages de la présente invention apparaîtront de façon encore plus évidente au vu des différents exemples de mise en oeuvre exposés ci-après.

*Exemple 1 :*

Dans un réacteur équipé d'un système de régulation de température et de pH et d'un système d'agitation avec hélice 3 pâles, on a introduit 14 000 g d'eau, et 630 g d'une solution aqueuse de silicate de sodium à 236 g/L en équivalent $SiO_2$ le rapport pondéral (Rp) $SiO_2/Na_2O$ du silicate de sodium utilisé étant de 3,46.

Après la mise en marche de l'agitation (250 tours par minute) le pied de cuve ainsi constitué a été chauffé à 95°C et le pH a été amené à 7,5, en 11 minutes, par ajout d'une solution aqueuse d'acide sulfurique à 80 g/L (débit moyen de 61 g par minute).

Une fois le pH de 7,5 atteint, on a procédé à une addition continue de 4 320 g d'une solution aqueuse de silicate de sodium (Rp=3,46) à 236 g/L en équivalent SiO2, à un débit constant de 48 grammes par minute (durée de l'addition : 90 minutes), en maintenant le pH du milieu à une valeur égale à 7,5 (à 0,1 unité de pH près), par addition dans le milieu d'une solution aqueuse d'acide sulfurique à 80 g/L avec un débit contrôlé en fonction de l'évolution mesurée pour le pH du milieu. En bilan, 4 770 g de la solution d'acide sulfurique ont été ajoutés dans le milieu, ce qui correspond à un débit moyen de 53 grammes de solution d'acide sulfurique ajoutés par minute.

Après les 90 minutes d'addition, on a arrêté l'addition de silicate et on a poursuivi une addition d'acide jusqu'à stabilisation du pH du mélange réactionnel à 3,4. On a effectué un mûrissement en laissant la solution sous agitation pendant 5 minutes.

La bouillie obtenue a ensuite été filtrée sur filtre plan, puis le gâteau obtenu a été délité mécaniquement à un pH égal 5, puis il a été séché par atomisation.

Les caractéristiques physico-chimiques de la silice sèche obtenue sont les suivantes :

pH de poudre : 5,5

Teneur en NaSO$_4$ : 0,2 % en masse (par rapport à la masse totale du matériau à l'état sec)

Surface spécifique CTAB : 160 m$^2$/g

Surface spécifique BET : 160 m$^2$/g

Perte au feu à 1000°C : 4,9 %

Teneur résiduelle en eau après un traitement de 2 heure à 1000°C : 2,6%

Reprise en eau (selon la définition de l'invention) : 5,5 %.

*Exemple 2 :*

Dans un réacteur équipé d'un système de régulation de température et de pH et d'un système d'agitation avec hélice 3 pâles, on a introduit 14 000 g d'eau, et 450 g d'une solution aqueuse de silicate de sodium à 236 g/L en équivalent $SiO_2$, le rapport pondéral (Rp) $SiO_2/Na_2O$ du silicate de sodium utilisé étant de 3,46.

Après la mise en marche de l'agitation (250 tours par minute) le pied de cuve ainsi constitué a été chauffé à 98°C et le pH a été amené à 7,5, en 11 minutes, par ajout d'une solution aqueuse d'acide sulfurique à 80 g/L (débit moyen de 61 g par minute).

Une fois le pH de 7,5 atteint, on a procédé à une addition continue de 3150 g d'une solution aqueuse de silicate de sodium (Rp=3,46) à 236 g/L en équivalent SiO2, à un débit constant de 35 grammes par minute (durée de l'addition : 90 minutes), en maintenant le pH du milieu à une valeur égale à 7,5 (à 0,1 unité de pH près), par addition dans le milieu d'une solution aqueuse d'acide sulfurique à 80 g/L avec un débit contrôlé en fonction de l'évolution mesurée pour le pH du milieu. En bilan, 3 510 g de la solution d'acide sulfurique ont été ajoutés dans le milieu, ce qui correspond à un débit moyen de 39 grammes de solution d'acide sulfurique ajoutés par minute.

Après les 90 minutes d'addition, on a arrêté l'addition de silicate et on a poursuivi une addition d'acide jusqu'à stabilisation du pH du mélange réactionnel à 3,4. On a effectué un mûrissement en laissant la solution sous agitation pendant 5 minutes.

La bouillie obtenue a ensuite été filtrée sur filtre plan, puis le gâteau obtenu a été délité mécaniquement à un pH égal 5, puis il a été séché par atomisation.

Les caractéristiques physico-chimiques de la silice sèche obtenue sont les suivantes :

pH de poudre : 5,2

Teneur en $NaSO_4$ : 0,3 % en masse (par rapport à la masse totale du matériau à l'état sec)

Surface spécifique CTAB : 164 $m^2$/g

Surface spécifique BET : 165 $m^2$/g

Perte au feu à 1000°C : 4,8 %

Teneur résiduelle en eau après un traitement de 2 heure à 1000°C : 2,6 %

Reprise en eau (selon la définition de l'invention) : 5,5 %.

### Exemple 3 :

Dans un réacteur équipé d'un système de régulation de température et de pH et d'un système d'agitation avec hélice 3 pâles, on a introduit 11 000 g d'eau, et 630 g d'une solution aqueuse de silicate de sodium à 236 g/L en équivalent $SiO_2$ le rapport pondéral (Rp) $SiO_2$/$Na_2O$ du silicate de sodium utilisé étant de 3,46.

Après la mise en marche de l'agitation (250 tours par minute) le pied de cuve ainsi constitué a été chauffé à 95°C et le pH a été amené à 7,5, en 11 minutes, par ajout d'une solution aqueuse d'acide sulfurique à 80 g/L (débit moyen de 61 g par minute).

Une fois le pH de 7,5 atteint, on a procédé à une addition continue de 4 320 g d'une solution aqueuse de silicate de sodium (Rp=3,46) à 236 g/L en équivalent SiO2, à un débit constant de 48 grammes par minute (durée de l'addition : 90 minutes), en maintenant le pH du milieu à une valeur égale à 7,5 (à 0,1 unité de pH près), par addition dans le milieu d'une solution aqueuse d'acide sulfurique à 80 g/L avec un débit contrôlé en fonction de l'évolution mesurée pour le pH du milieu. En bilan, 4 770 g de la solution d'acide sulfurique ont été ajoutés dans le milieu, ce qui correspond à un débit moyen de 53 grammes de solution d'acide sulfurique ajoutés par minute.

Après les 90 minutes d'addition, on a arrêté l'addition de silicate et on a poursuivi une addition d'acide jusqu'à stabilisation du pH du mélange réactionnel à 3,4. On a effectué un mûrissement en laissant la solution sous agitation pendant 5 minutes.

La bouillie obtenue a ensuite été filtrée sur filtre plan, puis le gâteau obtenu a été délité mécaniquement à un pH égal 5, puis il a été séché par atomisation.

Les caractéristiques physico-chimiques de la silice sèche obtenue sont les suivantes :

pH de poudre : 5,4

Teneur en $NaSO_4$ : 0,2 % en masse (par rapport à la masse totale du matériau à l'état sec)

Surface spécifique CTAB : 110 $m^2$/g

Surface spécifique BET : 126 $m^2$/g

Perte au feu à 1000°C : 5,1 %

Teneur résiduelle en eau après un traitement de 2 heure à 1000°C : 2,7%

Reprise en eau (selon la définition de l'invention) : 5,2 %.

**Exemple 4 :**

Dans un réacteur équipé d'un système de régulation de température et de pH et d'un système d'agitation avec hélice 3 pâles, on a introduit 14 000 g d'eau, et 450 g d'une solution aqueuse de silicate de sodium à 236 g/L en équivalent $SiO_2$, le rapport pondéral (Rp) $SiO_2/Na_2O$ du silicate de sodium utilisé étant de 3,46.

Après la mise en marche de l'agitation (250 tours par minute) le pied de cuve ainsi constitué a été chauffé à 95°C et le pH a été amené à 7,5, en 11 minutes, par ajout d'une solution aqueuse d'acide sulfurique à 80 g/L (débit moyen de 61 g par minute).

Une fois le pH de 7,5 atteint, on a procédé à une addition continue de 3 150 g d'une solution aqueuse de silicate de sodium (Rp=3,46) à 236 g/L en équivalent SiO2, à un débit constant de 35 grammes par minute (durée de l'addition : 90 minutes), en maintenant le pH du milieu à une valeur égale à 7,5 (à 0,1 unité de pH près), par addition dans le milieu d'une solution aqueuse d'acide sulfurique à 80 g/L avec un débit contrôlé en fonction de l'évolution mesurée pour le pH du milieu. En bilan, 3 500 g de la solution d'acide sulfurique ont été ajoutés dans le milieu, ce qui correspond à un débit moyen de 40 grammes de solution d'acide sulfurique ajoutés par minute.

Après les 90 minutes d'addition, on a arrêté l'addition de silicate et on a poursuivi une addition d'acide jusqu'à stabilisation du pH du mélange réactionnel à 3,4. On a effectué un mûrissement en laissant la solution sous agitation pendant 5 minutes.

La bouillie obtenue a ensuite été filtrée sur filtre plan, puis le gâteau obtenu a été délité mécaniquement à un pH égal 5, puis il a été séché par atomisation.

Les caractéristiques physico-chimiques de la silice sèche obtenue sont les suivantes :

pH de poudre : 5,4

Teneur en $NaSO_4$ 0,2 % en masse (par rapport à la masse totale du matériau à l'état sec)

Surface spécifique CTAB : 121 $m^2/g$

Surface spécifique BET : 131 $m^2/g$

Perte au feu à 1000°C : 5,4 %

Teneur résiduelle en eau après un traitement de 2 heure à 1000°C : 2,7%

Reprise en eau (selon la définition de l'invention) : 5,2 %.

**Exemple 5 :**

Dans un réacteur équipé d'un système de régulation de température et de pH et d'un système d'agitation avec hélice 3 pâles, on a introduit 950 litres d'eau, et 33 litres d'une solution aqueuse de silicate de sodium à 236 g/L en équivalent $SiO_2$, ie rapport pondéral (Rp) $SiO_2/Na_2O$ du silicate de sodium utilisé étant de 3,46.

Après la mise en marche de l'agitation (250 tours par minute) le pied de cuve ainsi constitué a été chauffé à 95°C et le pH a été amené à 7,5, en 11 minutes, par ajout d'une solution aqueuse d'acide sulfurique à 80 g/L (débit moyen de 219 litres par heure).

Une fois le pH de 7,5 atteint, on a procédé à une addition continue de 290 litres d'une solution aqueuse de silicate de sodium (Rp=3,46) à 236 g/L en équivalent SiO2 à un débit constant de 205 litre par heure (durée de l'addition : 85 minutes), en maintenant le pH du milieu à une valeur égale à 7,5 (à 0,2 unité de pH près), par addition dans le milieu d'une solution aqueuse d'acide sulfurique à 80 g/L avec un débit contrôlé en fonction de l'évolution mesurée pour le pH du milieu. En bilan, 375 litres de la solution d'acide sulfurique ont été ajoutés dans le milieu, ce qui correspond à un débit moyen de 265 litres de solution d'acide sulfurique ajoutés par heure.

Après les 85 minutes d'addition, on a arrêté l'addition de silicate et on a poursuivi une addition d'acide jusqu'à stabilisation du pH du mélange réactionnel à 3,4. On a effectué un mûrissement en laissant la solution sous agitation pendant 5 minutes.

La bouillie obtenue a ensuite été filtrée sur filtre plan, puis le gâteau obtenu a été délité mécaniquement à un pH égal 5, puis il a été séché par atomisation (atomiseur à buse).

Les caractéristiques physico-chimiques de la silice sèche obtenue sont les suivantes :

pH de poudre : 5,1

Teneur en NaSO$_4$ : 0,28 % en masse (par rapport à la masse totale du matériau à l'état sec)

Surface spécifique CTAB : 157 m$^2$/g

Surface spécifique BET : 165 m$^2$/g

Perte au feu à 1000°C : 5,4 %

Teneur résiduelle en eau après un traitement de 2 heure à 1000°C : 3,1%

Reprise en eau (selon la définition de l'invention) : 5,7 %.

**Revendications**

1.  Procédé de préparation d'une silice de précipitation à faible reprise en eau, comprenant les étapes successives consistant à :

    (a) réaliser un pied de cuve initial comprenant un silicate, la concentration en silicate dans ledit pied de cuve, exprimée en équivalent SiO$_2$, étant inférieure à 15 g/L ;
    (b) par addition d'un agent acidifiant, amener le pH du milieu jusqu'à une valeur comprise entre 7 et 8 ;
    (c) dans le milieu ainsi réalisé, effectuer l'addition simultanée d'un silicate et d'un agent acidifiant, les quantités respectives de silicate et d'agent acidifiant ajoutées au cours du temps étant spécifiquement choisies de façon à ce que, pendant toute la durée de l'addition :

    - le pH du milieu réactionnel reste compris entre 7 et 8 ;
    - la concentration en silicium dans le milieu, exprimée en équivalent SiO$_2$, reste inférieure ou égale à 35 g/L ;

    (d) ajouter un agent acidifiant au milieu obtenu à l'issue de l'étape (c), de façon à porter le milieu à un pH compris entre 3 et 6,5 ; et
    (e) filtrer la dispersion aqueuse de silice obtenue, puis sécher le gâteau de filtration réalisé à l'issu de la filtration.

2.  Procédé selon la revendication 1, **caractérisé en ce que** les silicates mis en oeuvre dans les étapes (a) et (c) sont des silicates alcalins.

3.  Procédé selon la revendication 1 ou selon la revendication 2, **caractérisé en ce que** les agents acidifiants mis en oeuvre dans les étapes (b), (c) et (d) sont choisis parmi l'acide sulfurique, l'acide chlorhydrique, l'acide nitrique, l'acide acétique, l'acide formique et l'acide carbonique.

4.  Procédé selon l'une quelconque des revendications 1 à 3, **caractérisé en ce que** le pied de cuve de l'étape (a) se présente sous la forme d'une solution aqueuse de silicate, de concentration, exprimée en équivalent SiO$_2$ inférieure ou égale à 10 g/L.

5.  Procédé selon l'une quelconque des revendications 1 à 4, **caractérisé en ce que** l'agent acidifiant de l'étape (b) est introduit sous la forme d'une solution aqueuse de normalité comprise entre 0,25 N et 8 N.

6.  Procédé selon l'une quelconque des revendications 1 à 5, **caractérisé en ce que** l'agent acidifiant de l'étape (b) est l'acide sulfurique, introduit sous la forme d'une solution aqueuse de concentration comprise entre 10 g/L et 350 g/L.

7.  Procédé selon l'une quelconque des revendications 1 à 6, **caractérisé en ce que** l'addition simultanée du silicate et de l'agent acidifiant de l'étape (c) est réalisée par addition continue de silicate dans le milieu, le pH étant régulé au cours de l'addition par introduction d'agent acidifiant lorsque le pH du milieu devient supérieur à une valeur de contrôle comprise entre 7 et 8.

8.  Procédé selon l'une quelconque des revendications 1 à 6, **caractérisé en ce que** l'addition simultanée du silicate et de l'agent acidifiant de l'étape (c) est réalisée par addition continue d'agent acidifiant dans le milieu, le pH étant régulé au cours de l'addition par introduction de silicate lorsque le pH du milieu devient inférieur à une valeur de contrôle comprise entre 7 et 8.

9. Procédé selon l'une quelconque des revendications 1 à 6, **caractérisé en ce que** l'addition simultanée du silicate et de l'agent acidifiant de l'étape (c) consiste en une addition continue à la fois d'agent acidifiant et de silicate, avec des concentrations et des débits calculés de façon à ce que, durant toute la durée de l'addition, le pH du milieu reste compris entre 7 et 8.

10. Procédé selon l'une quelconque des revendications 1 à 9, **caractérisé en ce que** le silicate introduit lors de l'addition simultanée de l'étape (c) est sous la forme d'une solution aqueuse de concentration comprise entre 10 g/L et 360 g/L.

11. Procédé selon l'une quelconque des revendications 1 à 10, **caractérisé en ce que** l'agent acidifiant introduit lors de l'addition simultanée de l'étape (c) est sous la forme d'une solution aqueuse de normalité comprise entre 0,25 Net8N.

12. Procédé selon l'une quelconque des revendications 1 à 11, **caractérisé en ce que** la durée de l'addition de l'étape (c) est comprise entre 15 et 300 minutes.

13. Procédé selon l'une quelconque des revendications 1 à 12, **caractérisé en ce qu'**on introduit un composé d'aluminium dans le milieu, à la fin de l'étape (c), et/ou entre l'étape (c) et l'étape (e).

14. Procédé selon l'une quelconque des revendications 1 à 13, **caractérisé en ce que** l'étape (d) est mise en oeuvre, et **en ce que** l'agent acidifiant de ladite étape (d) est introduit dans le milieu sous la forme d'une solution aqueuse de normalité comprise entre 0,25 et 8 N.

15. Procédé selon l'une quelconque des revendications 1 à 14, **caractérisé en ce que** les étapes (a), (b), (c) et (d) sont conduites à une température comprise entre 90 et 100°C.

16. Procédé selon l'une quelconque des revendications 1 à 15, **caractérisé en ce que** la dispersion aqueuse de silice obtenue à l'issue de l'étape (d) est soumise à une étape de mûrissement, préalablement à l'étape (e).

17. Procédé selon l'une quelconque des revendications 1 à 16, **caractérisé en ce que** l'étape (e) comprend une opération de délitage du gâteau de précipitation.

**Claims**

1. A process for preparing a low water-uptake precipitated silica, comprising the following successive steps consisting in :

   (a) producing an initial feedstock comprising a silicate, the Silicate concentrations in the feedstock, expressed in $SiO_2$ equivalent, being less than 15 g/l;
   (b) by adding an acidifying agent, bringing the pH of the medium to a value of between 7 and 8 ;
   (c) in the resulting medium, simultaneously adding a silicate and an acidifying agent, the respective amounts of added silicate and acidifying agent over time being specifically selected such that, throughout the addition:

      - the pH of the reaction medium remains between 7 and 8;
      - the silicon concentration in the medium, expressed in $SiO_2$ equivalent, remains less than or equal to 35 g/l;

   (d) adding an acidifying agent to the medium resulting from step (c), so as ta bring the medium at a pH of between 3 and 6.5; and
   (e) filtering the resulting aqueous silica dispersion, then drying the filter cake obtained at the end of the filtering step.

2. The process of claim 1, wherein the silicates used in steps (a) and (c) are alkali silicates.

3. The process of claim 1 or 2, wherein the acidifying agents used in steps (b) , (c) and (d) are selected among sulfuric acid, hydrochloric acid, nitric acid, acetic acid, formic acid and carbonic acid.

4. The process of anyone of claims 1 to 3, wherein the feedstock of step (a) is in the form of an aqueous silicate solution, having a concentration, expressed in $SiO_2$ equivalent, of less than or equal to 10 g/l.

5. The process of anyone of claim 1 to 4, wherein the acidifying agent of step (b) is introduced in the form of an aqueous solution having a normality of between 0.25 N and 8 N.

6. The process of anyone of claims 1 to 5, wherein the acidifying agent of step (b) , is sulfuric acid, introduced in the form of an aqueous solution having a concentration of between 10 g/l and 350 g/l.

7. The process of anyone of claims 1 to 6, wherein the simultaneous addition of the silicate and acidifying agent of step (c) is carried out by continuously adding silicate to the medium, the pH being adjusted duping the addition by introducing acidifying agent if the pH of the medium becomes greater than a given control value, of between 7 and 8.

8. The process of anyone of claims 1 to 6, wherein the simultaneous addition of the silicate and acidifying agent of step (c) is carried out by continuously adding silicate to the medium, the pH being adjusted during the addition by introducing silicate if the pH of the medium becomes less than a test value of between 7 and 8.

9. The process of anyone of claims 1 to 6, wherein the simultaneous addition of the silicate and acidifying agent of step (c) consists in continuously adding both acidifying agent and silicate, with concentrations and flow rates calculated such that, throughout the addition, the pH of the medium remains between 7 and 8.

10. The process of anyone of claims 1 to 9, wherein the silicate that is introduced during the simultaneous addition of step (c) is in the form of an aqueous solution having a concentration of between 10 g/l and 360 g/l.

11. The process of anyone of claims 1 to 10, wherein the acidifying agent that is introduced during the simultaneous addition of step (c) is in the form of an aqueous solution having a normality of between 0.25 N and 8 N.

12. The process of anyone of claims 1 to 11, wherein the addition of step (c) last between 15 and 300 minutes.

13. The process of anyone of claims 1 to 12, wherein an aluminum compound is introduced two the medium at the end of step (c), and/or between step (c) and step (e) .

14. The process of anyone of claims 1 to 13, wherein step (d) is used, and in that the acidifying agent from step (d) is introduced to the medium in the form of an aqueous solution having a normality of between 0.25 N and 8.

15. The process of anyone of claims 1 to 14, wherein steps (a), (b) (c) and (d) are carried out at a temperature of between 90 and 100 °C.

16. The process of anyone of claims 1 to 15, wherein the aqueous silica dispersion resulting from step (d) is subjected to a maturation step, prior to step (e).

17. The process of anyone of claims 1 to 16, wherein step (e) comprises a process of splitting the precipitate cake.

**Patentansprüche**

1. Verfahren zur Herstellung einer Fällunaskieselsäure mit geringer Wasseraufnahme mit den aufeinanderfolgenden Schritten, die darin bestehen:

(a) eine anfängliche Küvettenbasis mit einem Silikat herzustellen, wobei die Silikatkönzentration in der Küvettenbasis, als $SiO_2$-Äquivalent ausgedrückt, geringer ist als 15 g/l;
(b) durch Zugabe eines Ansäucrybgsnuttels den pH Wert des Mediums auf einen Wert zwischen 7 und 8 zu bringen;
(c) in dem so hergestellten Medium die gleichzeitige Zugabe eines Silikats und eines Ansäuerungsmittels durchzuführen, wobei die jeweiligen im Zeitverlauf zugegebenen Mengen an Silikat und Ansäuerungsmittel spezifisch so gewählt werden, dass während der ganzen Dauer der Zugabe:

- derpH-Wert des Reaktionsmediums zwischen 7 und 8 bleibt;

- die Siliziumkonzentration im Medium, als SiO$_2$-Äquivalent ausgedrückt, unterhalb oder gleich 35 g/l bleibt;

(d) ein Ansäuerungsmittel zu dem am Ende von Schritt (c) erhaltenen Medium zuzugeben, um das Medium auf einen pH-Wert zwischen 3 und 6,5 zu dringen; und

(e) die erhaltene wässerige Kieseisäuredispersion zu filtrieren, dann den am Ende der Filtration hergestellten Fillerkuchen zu trocknen.

2. Verfahren nach Anspruch 1, **dadurch gekennzeichnet, dass** die in den Schritten (a) und (c) eingesetzten Silikate alkalische Silikate sind.

3. Verfahren nach Anspruch 1 oder nach Anspruch 2, **dadurch gekennzeichnet, dass** die in den Schritten (b). (c) und (d) eingesetzten Ansäuerungsmittel aus Schwefelsäure, Salzsäure, Salpetersäure, Essigsäure, Ameisensäure und Kohlensäure ausgewählt sind.

4. Verfahren nach einem der Ansprüche 1 bis 3, **dadurch gekennzeichnet, dass** die Küvettenbasis des Schritts (a) in Form einer wässerigen Silikatlösung mit einer Konzentration, in SiO$_2$-Äquivalent ausgedrückt, die kleiner oder gleich 10 g/l ist, vorliegt.

5. Verfahren nach einem der Ansprüche 1 bis 4, **dadurch gekennzeichnet, dass** das Ansäuerungsmittel des Schritts (b) in Form einer wässerigen Lösung mit einer Norrmalität zwischen 0,25 N und 8 N eingeführt wird.

6. Verfahren nach einem der Ansprüche 1 bis 5, **dadurch gekennzeichnet, dass** das Ansäuerungsmittel des Schritts (b) Schwefelsäure ist, die in Form einer wässerigen Lösung mit einer Konzentration zwischen 10 g/l und 350 g/l eingeführt wird.

7. Verfahren nach einem der Ansprüche ! 1 bis 6, **dadurch gekennzeichnet, dass** die gleichzeitige Zugabe des Silikats und des Ansäuerungsinittels des Schritts (c) durch kontinuierliche Zugabe von Silikat in das Medium ausgeführt wird, wobei der pH-Wert im Verlauf der Zugabe durch Einführung eines Ansäuerungsmittels geregelt wird, wenn der pH-Wert des Mediums höher wird als ein Kontrollwert zwischen 7 und 8.

8. Verfahren nach einem der Ansprüche 1 bis 6, **dadurch gekennzeichnet, dass** die gleichzeitige Zugabe des Silikats und des Ansäuerungsmittels des Schritts (c) durch kontinuierliche Zugabe eines Ansäuerungsmittels in das Medium ausgeführt wird, wobei der pH-Wert im Verlauf der Zugabe durch Beinführung von Silikat geregelt wird, wenn der pH-Wert des Mediums geringer wird als ein Kontrollwert zwischen 7 und 8.

9. Verfahren nach einem der Ansprüche 1 bis 6, **dadurch gekennzeichnet, dass** die gleichzeitige Zugabe des Silikats un des Ansäuerungsmittels des Schritts (c) in einer kontinuierlichen Zugabe sowohl eines Ansäuerybgsnuttels als auch von Silikat mit Konzentrationen und Mengen besteht, die so berechnet werden, dass während der gesamten Dauer der Zugabe der pH-Wert des Mediums zwischen 7 und 8 bleibt.

10. Verfahren nach einem der Ansprüche 1 bis 9, **dadurch gekennzeichnet, dass** das bei der gleichzeitige Zugabe des Schritts (c) eingeführte Silikat in Form einer wässerigen Lösung mit einer Konzentration zwischen 10 g/l und 360 g/l vorliegt.

11. Verfahren nach einem der Ansprüche 1 bis 10, **dadurch gekennzeichnet, dass** das bei der gleichzeitigen Zugabe des Schritts (c) eingeführte Ansäuerungsmittel in Form einer wässerigen Lösung mit einer Normalität zwischen 0,25 N und 8 N vorliegt.

12. Verfahren nach einem der Ansprüche 1 bis 11, **dadurch gekennzeichnet, dass** die Dauer der Zugabe des Schritts (c) zwischen 15 und 300 Minuten liegt.

13. Verfahren nach einem der Ansprüche 1 bis 12, **dadurch gekennzeichnet, dass** eine Aluminiumverbindung in das Medium am Ende des Schritts (c) und/oder zwischen Schritt (c) und Schritt (e) eingeführt wird.

14. Verfahren nach einem der Ansprüche 1 bis 13, **dadurch gekennzeichnet, dass** der Schritt (d) ausgeführt wird und dass das Ansäuerungsmittel des Schritts (d) in das Medium in Form einer wässerigen Lösung mit einer Normalität zwischen 0,25 und 8 N eingeführt wird.

15. Verfahren nach einem der Ansprüche 1 bis 14, **dadurch gekennzeichnet, dass** die Schritte (a), (b), (c) und (d) bei einer Temperatur zwischen 90 und 200 °C durchgeführt werden.

16. Verfahren nach einem der Ansprüche 1 bis 15, **dadurch gekennzeichnet, dass** die wässerige Kieselsäuredispersion, die am Ende von Schritt (d) erhalten wird, vor dem Schritt (e) einem Reifungsschritt unterzogen wird.

17. Verfahren nach einem der Ansprüche 1 bis 16, **dadurch gekennzeichnet, dass** der Schritt (e) einen Spaltungsvorgang des Fäliungskuchens umfasst.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2356596
- EP 0280623 A
- WO 950127 A
- WO 9509128 A
- FR 1111969 A

**Littérature non-brevet citée dans la description**

- *Angewandte Chemie,* 1960, vol. 72, 744
- *Journal of the American Chemical Society,* Février 1938, vol. 60, 309